# EUROPEAN PATENT SPECIFICATION

(11) **EP 2 468 090 B1**
(45) Date of publication and mention of the grant of the patent: **26.03.2014**
(21) Application number: 10196195.1
(22) Date of filing: 21.12.2010
(51) Int. Cl.: A01G 7/06, A01G 7/04, A01G 17/00, A01H 3/02, A01H 4/00, F21K 99/00, H05B 33/00, H05B 33/08

(54) **Method and means for acclimatizing seedlings for outdoor life**
Verfahren und Mittel zur Akklimatisierung von Stecklingen für das Leben draußen
Procédé et moyens pour acclimater des semis à la vie en extérieur

(43) Date of publication of application: 27.06.2012
(60) Divisional application: 13192115.7
(73) Proprietor: Valoya Oy, 00200 Helsinki (FI)
(72) Inventor: Aikala, Lars, 02520, Lapinkylä (FI); Kivimäki, Ilkka, 00150, Helsinki (FI)
(74) Representative: Väänänen, Mikko Kalervo

(56) References cited:
- WO-A1-2007/049962
- WO-A1-2009/131008
- WO-A1-2010/053341
- US-A1- 2008 120 736
- US-A1- 2010 259 190
- KIM JONG JIN ET AL: "Effects of UV-B radiation and water stress on hardening phase growth of container-grown Betula platyphylla seedlings", JOURNAL OF KOREAN FORESTRY SOCIETY, vol. 87, no. 4, December 1998 (1998-12), pages 601-610, XP009148772, ISSN: 0445-4650
- CLOSE D. ET AL.: "The physiological basis of containerized tree seedling "transplant shock": a review", AUSTRALIAN FORESTRY, vol. 68, no. 2, 2005, pages 112-120, XP002638857,
- HOFFMANN SILKE: "The effect of UV-radiation on colours of leaves and flowers of ornamental plants", GARTENBAUWISSENSCHAFT, vol. 64, no. 2, March 1999 (1999-03), pages 88-93, XP009148682, ISSN: 0016-478X
- FISCHER A L: "LEDs in the greenhouse", PHOTONICS SPECTRA LAURIN PUBLISHING CO. INC. USA, vol. 42, no. 7, July 2008 (2008-07), page 78, XP009151843, ISSN: 0731-1230
- LIANG MA ET AL: "Investigation of Eu-Mn energy transfer in A3MgSi2O8:Eu<2+>, Mn<2+> (A=Ca,Sr,Ba) for light-emitting diodes for plant cultivation", APPLIED PHYSICS LETTERS AMERICAN INSTITUTE OF PHYSICS USA, vol. 93, no. 14, 6 October 2008 (2008-10-06), XP000002658425, ISSN: 0003-6951
- SCHUERGER ANDREW C ET AL: "Anatomical features of pepper plants (Capsicum annuum L.) grown under red light-emitting diodes supplemented with blue or far-red light", ANNALS OF BOTANY, ACADEMIC PRESS, LONDON, GB, vol. 79, no. 3, 1 January 1997 (1997-01-01), pages 273-282, XP002574159, ISSN: 0305-7364, DOI: 10.1006/ANBO.1996.0341

## Description

### TECHNICAL FIELD OF INVENTION

The invention relates to a method for acclimatizing seedlings for outdoor life. More particularly the invention relates to lighting method that can be used to treat a seedling in a growth chamber or greenhouse prior to the introduction of the seedling to outdoors.

### BACKGROUND

Industrially produced tree seedlings are typically grown in growth chambers or greenhouses at early stages of their life. When the seedlings reach a certain age or size, they are then subsequently planted outdoors in accordance with the prior art. The move out-doors is a very shocking experience to the plants. Among other things, the "transplantation shock" to the plant from moving outdoors comes from the difference in the light spectrum inside (the greenhouse or laboratory growth chamber conditions) and outside. In order to avoid this plants are often kept under shading curtains for a few weeks in order to limit the direct exposure to sunlight. This period causes additional work and investment to the grower and delays the maturing of the harvest.

The shock to the plants is caused by the abrupt exposure to the Sun, as the light they have received in the greenhouse or growth chamber has had a limited light spectrum compared to the outdoors and the light spectrum of the Sun. The light conditions indoors is comprised of Sun light, typically filtered through the greenhouse glass or polycarbonate, and in most cases also additional artificial light from high-pressure sodium lamps during hours with less natural light. Fluorescent tubes are often used in growth chambers. Special LED based lights are also getting more common, which may/will replace HPS lights and fluorescent lights in the future.

Tree seedlings have thus been reported to suffer from a 'transplant shock', i.e. seedling mortality or impaired growth, after they have been introduced outdoors. This effect is reviewed in the article "The physiological basis of containerised tree seedlings 'transplant shock': a review" by Close et al. which is cited here as reference.

US 2008/0120736 describes a method of illumination of plants in the PAR (Photosynthetically active radiation) and UV-A and UV-B, or infrared regions of the spectrum. The UV illumination is alleged to increase insect resistance, immune response, enhance pigmentation and aroma, and alter plant architecture such as shape, flower number and volume and trichome density. This document is also cited here as reference.

It is further known from "Photobiology of higher plants", p. 28, that UV radiation stimulates the production of phenolic compounds protective of excess radiation. Also it is known from "Photobiology of higher plants", p. 136, that many plants in high light environments increase the reflectance of their leaves by acquiring a coat of leaf hairs or wax, as a means of external photoprotection. This document is also cited here as reference. These two phenomena are well known among professional plant photobiologists, but they have not been utilized in any practical way.

EP 0364952 A2 shows a method of irradiating seeds with UV. The viability of seeds is tested with this method, as non-viable seeds cause fluorescence of sinapine. This document is also cited here as reference.

In summary, it appears that in the prior art UV enhancement of plants is known to provide a variety of photomorphogenic and other effects. In addition UV illumination is used as a method to detect the viability of seeds.

The methods of the prior art have considerable shortcomings. The detection of seed viability is essentially useless if the seedling eventually dies of the aforementioned transplantation shock. Furthermore, photomorphogenical enhancement of plants by increasing their size or flower number is also ineffective in view of the eventual outcome, if the seedling does not survive the transplantation shock. Using shading by curtains is uneconomical as it disproportionately increases the sorting of the seedlings done by the plant producer. This is because transporting the plants under the shades and removing them from under the shades also adds one additional costly work phase to the seedling producer.

"Effects of UV-B Radiation and Water Stress on Hardening Phase Growth of Container-Grown Betula platyphylla Seedlings", Jong Jin Kim and Sung Gak Hong Jour. Korean For. Soc. 87(4). 601 - 610. 1998, is cited here as reference.

### SUMMARY

The invention under study is directed towards a system and a method for effectively treating tree seedlings against the transplantation shock prior to their entry to outdoors.

First objective of the invention is to present this treatment to the seedling in a manner that takes place in a short period of time, thereby minimising the sorting of seedlings that the plant grower needs to manage. Further objective is to provide a treatment for transplantation shock that is effective on northern and southern latitudes, or any different latitudes, as the amount of sunlight, and therefore the requirements for successful transplantation shock treatment might vary with latitude.

Another objective of the invention is to provide a transplantation shock treatment that can be applied to seedlings grown in a dark growth chamber, or in shadow due to e.g. stacking, or in any growth environment characterized by the absence of natural sunlight.

Third objective of the invention is to provide a transplantation shock treatment that can be applied to plants housed in a greenhouse wherein the seedlings receive some natural sunlight and artificial light is used to supplement this natural light for transplantation shock treatment. An even further objective of the invention is to provide artificial light that can be applied during the night in a greenhouse to seedlings and/or plants for transplantation shock treatment.

Also disclosed is a light by which the plants can be prepared for the outdoor conditions, by giving them certain wavelengths of light they do not currently receive from the light in the greenhouse or growth chambers. The light of the invention can be applied in smaller doses during the major part of nursing of the seedlings or as a "sun-shock" period in the end of the indoors nursing period. By giving the seedlings light from the invention, they are prepared to Sun light and do not need to spend a few weeks under the sunshade curtains.

In one embodiment of the invention, the growth chamber lighting device emits radiation that gives the plants the parts of radiation they have not received during their growth period. The key spectral areas of the device are the UV-A (315-400 nm), UV-B (280-315 nm) and as well as the violet and blue areas (400-500 nm), as well as red and far red areas (600-800 nm). In some embodiments of the invention, the device may also contain green and yellow areas of the spectrum (500-600 nm).

The invention thus improves the growth cycle of tree seedlings, enhances the proportion of viable seedlings and eliminates one work phase in the growth process, thus improving the economics of seedling cultivation and growth.

A method of treating plants is in accordance with the invention as claimed in claim 1.

Use of artificial light to treat transplantation shock in plants is in accordance with the invention as claimed in claim 12.

According to one aspect of the invention, the tree seedlings are grown in a greenhouse with transparent or semi-transparent walls and ceiling, typically made of polycarbonate or any other plastic and/or glass. In this embodiment artificial light is shone on the seedlings from LEDs that supplement the natural spectrum that transmits through the walls and ceilings of the greenhouse. As we know that high energy photons get cut off by some materials in greenhouse walls or ceilings, the artificial light typically emits UV photons. The compound spectrum of the natural light and the artificial light is arranged to treat the seedling against transplantation shock. In further aspects of the invention the artificial light is used during the night or when the Sun is low on the horizon to treat the transplantation shock.

According to another aspect of the invention, the tree seedlings are housed in a dark growth chamber where there is no natural sunlight. In embodiments where the sole source of light for the plant is artificial light, the transplantation shock treatment can be arranged to be conducted by the primary light source at some stage in the cultivation period of the seedling, or by a special light source that will be used at different times. The special light source providing the transplantation shock treatment may be integrated in the primary growth light device. The spectrum of the light for transplantation shock treatment needs to form a preferred compound spectrum with primary light source when it is in use, and/or form the entire transplantation shock treatment spectrum when no other lights are in use.

Some or all of the aforementioned advantages of the invention are accrued when the transplantation shock treatment is adjusted so that it interferes with the growth of the plant as little as possible.

In addition and with reference to the aforementioned advantage accruing embodiments, the best mode of the invention is considered to be the provision of small doses of high energy UV pulses to tree seedlings housed in a chamber with very limited access to sunlight.

### BRIEF DESCRIPTION OF THE DRAWINGS

In the following the invention will be described in greater detail with reference to exemplary embodiments in accordance with the accompanying drawings, in which
Figure 1 demonstrates an embodiment of the inventive method of treating plants for transplantation shock as a flow diagram.
Figure 2 demonstrates an embodiment 20 of the transplantation shock treatment of the invention when used in a greenhouse as a block diagram.
Figure 3 demonstrates an embodiment 30 of the transplantation shock treatment of the invention when used in a growth chamber as a block diagram.
Figure 4 demonstrates an embodiment 40 of the inventive method of treating plants in a greenhouse for transplantation shock as a flow diagram.
Figure 5 demonstrates an embodiment 50 of the inventive method of treating plants in a growth chamber with limited or no sunlight for transplantation shock as a flow diagram.
Figure 6 demonstrates an embodiment 60 of preferable LED spectra used in accordance with the invention that have been built and tested by the applicant.

Some of the embodiments are described in the dependent claims.

### DETAILED DESCRIPTION OF EMBODIMENTS

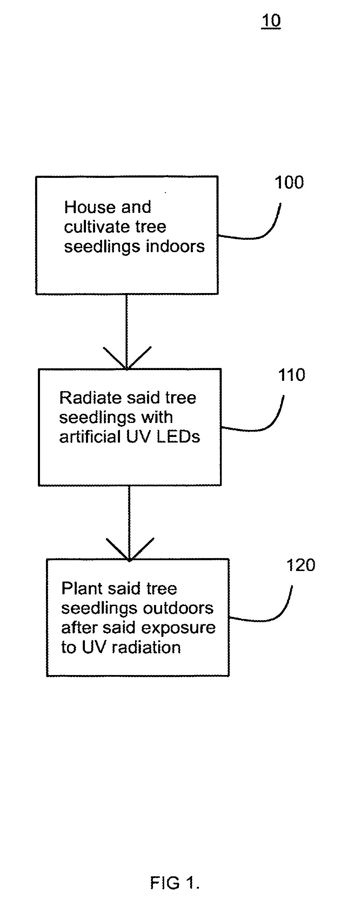

Figure 1 shows a method of treating plants against transplantation shock as a flow diagram 10. Typically the said plants are tree seedlings and are housed indoors at an early stage of their life. For a tree seedling the planting event is especially important as any injury in this phase might mean years of belated or inhibited growth, or outright early death to the tree seedling, amounting to substantial economic loss to the grower. The method of the invention can in principle be applied with all tree seedlings, but is especially suited for treating any of the following species against transplantation shock: Oak, acacia, pine, birch, maple, sequoia, redwood, eucalyptus, bamboo, palm, spruce, aspen, alder, linden, cypress, and/or any other tree species that is cultivated indoors in phase 100. In phase 110 the said tree seedlings are exposed to artificial UV light indoors prior to outdoor life. The incident UV light is produced by light emitting diodes (LEDs) in phase 110. In one embodiment the said artificial light is applied in small doses during the major part of nursing of the seedlings. In other embodiments a single period and/or a pulse in the end of the in-doors nursing period is used.

In some embodiments the said artificial light is applied in any of the following bands: UV-A (315-400 nm), UV-B (280-315 nm), violet and blue areas (400-500 nm), red and far red areas (600-800 nm) and/or green and yellow areas of the spectrum (500-600 nm). In some embodiments of the invention the light device is any of the following: a Light Emitting Diode (LED), AlInGaP red and AlInGaN green and/or blue HB-LEDs, a light wavelength up-conversion phosphorescing material which is deposited in direct proximity of the LED chip, europium-cerium co-doped BaₓSr_{y}ZnS₃ phosphorescing materials and/or cerium doped lanthanide oxide sulfides in direct proximity of the LED chip, and/or a wavelength up-conversion device by means of at least one semiconductor quantum dot, which is placed near the LED.

In this application "phosphor" is construed to refer to any phosphorescing material, which can be for example element phosphor, but it is not limited to only the element phosphor. Subscripts x and y denote numerical variables in a chemical formula in this application.

It should also further be noted that the embodiment 10 can be readily permuted and/or combined with any of the embodiments 20, 30, 40, 50 and/or 60 and be used to create any of the embodiments 20, 30, 40, 50 and/or 60.

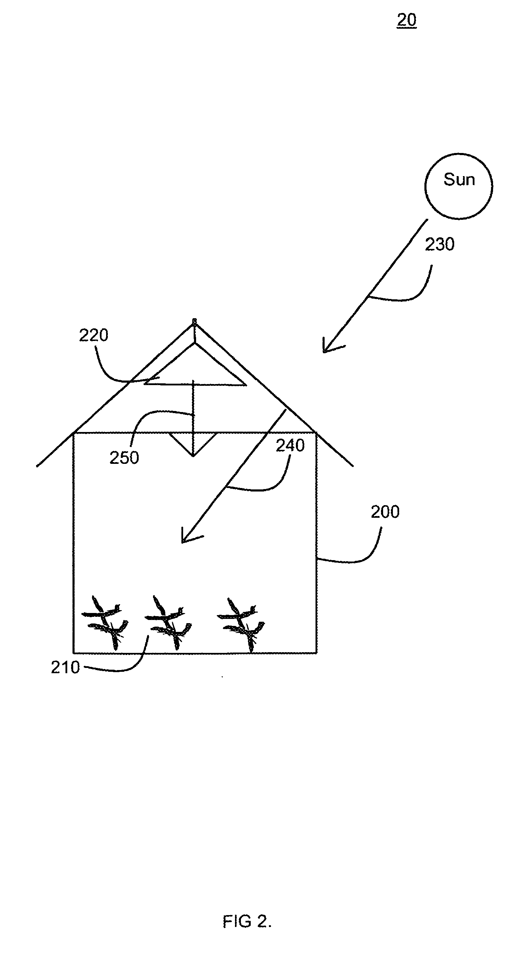

Figure 2 shows an embodiment where the inventive treatment is administered in a greenhouse 200. The greenhouse 200 has typically transparent walls, which are in some embodiments made of glass or plastic or a like transparent material. These materials typically block the high energy UV with wavelengths of 300- 400 nm or less from entering into the greenhouse, resulting to a modification in the spectrum from sunlight 230 to filtered sunlight 240. At least one tree seedling 210 is grown in the greenhouse in accordance with the invention. The artificial light 220 is typically physically attached to a location from which it has the maximum exposure and coverage of tree seedlings 210.

In some embodiments the said artificial light spectrum 250 combined with the spectrum of Sun light transparent through greenhouse walls or ceilings 230 amounts to a combined sum spectrum similar to the solar spectrum observed on the ground of the Earth. This acclimatizes the seedlings to outdoor life in preferable embodiments of the invention.

In some embodiments of the invention the green and yellow photons 500-600 nm are omitted from the artificial light 220 and its spectrum 250. In some embodiments of the invention the greenhouse walls and ceilings might comprise a filter for 500-600 nm light, because this band is not as photosynthetically active as blue or red band as plants reflect green light, and as this band might create unwanted heat. Green light is important to plants in other purposes, for example the plants derive a lot of photomorphogenetic information from green light, and its spectral ratios with other bands. Therefore in a preferred embodiment of the invention, there is green light present in the spectrum administered to treat transplantation shock, but this band of the spectrum has a smaller relative intensity to blue and red bands than in the spectrum of sunlight. In some embodiments of the invention even when the objective is to otherwise create a spectrum similar to the Sun indoors, the relative intensity of the 500-600 nm band is deliberately left smaller than in the sunlight spectrum.

The light device 220 is any of the following: a Light Emitting Diode (LED), AlInGaP red and AlInGaN green and/or blue HB-LEDs, a light wavelength up-conversion phosphorescing material which is deposited in direct proximity of the LED chip, europium-cerium co-doped BaₓSr_{y}ZnS₃ phosphorescing materials and/or cerium doped lanthanide oxide sulfides which is deposited in direct proximity of the LED chip, and/or a wavelength up-conversion device by means of at least one semiconductor quantum dot, which is placed near the LED. Suffixes x and y denote variables in the chemical formula of the compound. Furthermore in some embodiments of the invention the light device 220 may be equipped with any of the following phosphorescing materials expressed with the following chemical formula:
- MAISiN₃X (where in M is a Metal such as Ca, Sr, Ba and X is rare earth element such as Eu in any various ratios and combinations, or X is Mn in any various ratios and combinations),
- MMgSiOX (where in M is a Metal such as Ca, Sr, Ba and X is rare earth element such as Eu in any various ratios and combinations, or X is Mn in any various ratios and combinations).

In some embodiments the light device 220 is arranged to transmit a different spectrum at night than during the day. In some embodiments the spectrum is arranged to be changed dynamically with the time of day or season (i.e. date) or both in accordance with the invention.

It should also further be noted that the embodiment 20 can be readily permuted and/or combined with any of the embodiments 10, 30, 40, 50 and/or 60 and be used to create any of the embodiments 10, 30, 40, 50 and/or 60.

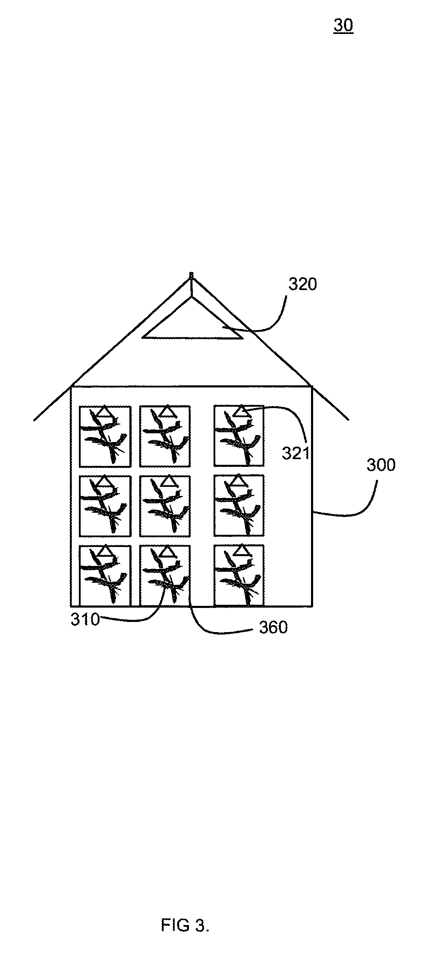

In figure 3 the tree seedlings are housed in at least one growth chamber 360. The growth chambers 360 are typically inside a building 300, and the growth chambers 360 are stacked to save space and cost for the grower. In some embodiments the growth chambers are transparent and the building is a transparent greenhouse as explained before, in some embodiments the building 300 is opaque, in some embodiments the growth chamber sides are made of opaque material in accordance with the invention.

In some embodiments where the growth chamber sides 360 as well as the building 300 are of transparent material there is only one or few light sources 320 for several tree seedlings. In other embodiments where all or some sides of the growth chamber are opaque light sources 321 may be placed closer to, or within the growth chambers 360 themselves to ensure sufficient treatment against transplantation shock.

It should also further be noted that the embodiment 30 can be readily permuted and/or combined with any of the embodiments 10, 20, 40 and/or 50 and be used to create any of the embodiments 10, 20, 40, 50 and/or 60.

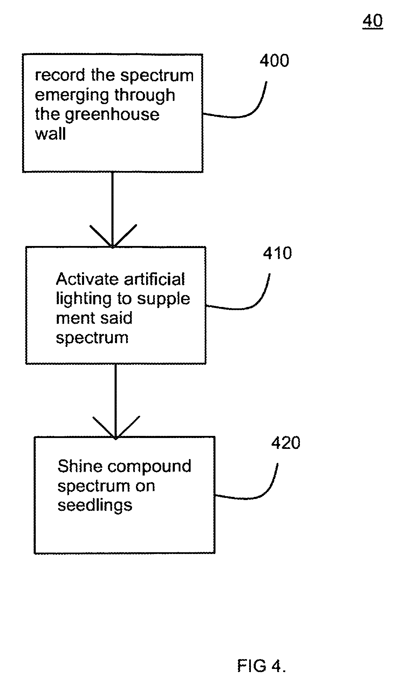

Figure 4 shows the treatment method embodiment used in the setup of figure 2 as a flow diagram. In phase 400 the spectrum emerging through the greenhouse walls and/or ceiling is recorded, for example with a spectrometer. This spectrum is supplemented by artificial light in phase 410. In many embodiments of the invention the artificial light of phase 410 is primarily UV light. This is because the high energy component typically in the UV has been reflected by the walls and/or ceilings of the greenhouse in some embodiments. In phase 420 the compound spectrum is shone on the tree seedlings.

In some embodiments the artificial lighting supplements the spectrum differently, depending what time it is and how much sunlight is available. For example during the night the artificial light can be used to produce the whole spectrum, which in some embodiments resembles sunlight spectrum.

It should also further be noted that the embodiment 40 can be readily permuted and/or combined with any of the embodiments 10, 20, 30, 50 and/or 60 and be used to create any of the embodiments 10, 20, 30, 50 and/or 60.

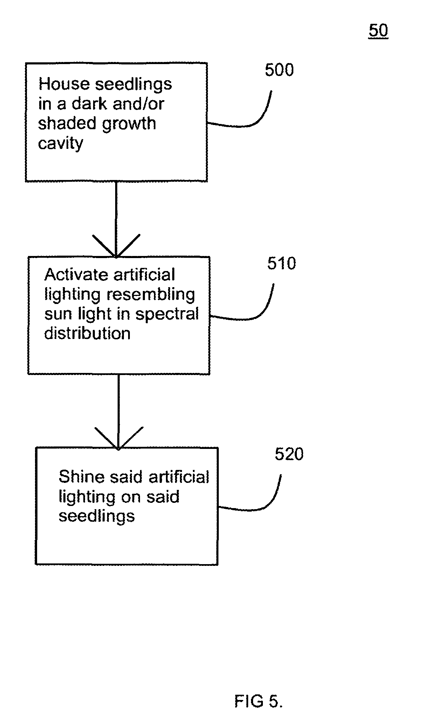

Figure 5 shows an embodiment 50 of the method that is used with the setup of figure 3 in some embodiments. The said tree seedlings are predominantly exposed to the artificial light spectrum, as seedlings are housed in a dark or shaded growth cavity 360 in phase 500.

In phase 510 the artificial lighting produces a spectrum that resembles sunlight, as the artificial light is nearly the sole source of light. In some embodiments the artificial light produces short pulses of UV from a location that is very close to the seedling to acclimatize the seedlings to outdoor life in phase 520. This is preferable in some embodiments of the invention as it minimizes the energy used in illumination and a great majority of the high UV photons intended to reach the seedling do reach the seedling, rather than being shined off target.

It should also further be noted that the embodiment 50 can be readily permuted and/or combined with any of the embodiments 10, 20, 30 and/or 40 and be used to create any of the embodiments 10, 20, 30, 40 and/or 60.

It should be noted that the artificial UV light generated to overcome transplantation shock can be arranged in various device configurations. In one embodiment the artificial UV light can be a LED light that emits solely or mainly in the UV-B band in accordance with the invention. In other embodiments the UV light is integrated to and with other light emitting devices, such as LEDs, that may emit in any of the following bands: UV, visible light, far red band (700-800 nm), infra red band (800nm+).

In some embodiments the light is produced by electroluminence or by phosphorence or both in accordance with the invention. For example, in one embodiment the UV light is produced by electroluminence and the light in the visible or IR band is produced by phosphor or phosphorescing material in the proximity of the UV light that absorbs the UV light and then emits light of longer wavelengths. In this phosphorence based embodiment it is possible to adjust the ratio of the intensities of UV emission and visible-infrared emission by adjusting the type and quantity of the phosphorescing material when the light device is manufactured.

Each LED may have one or more emission peaks in accordance with the invention both in the UV and in the visible, far red and/or infrared bands in accordance with the invention.

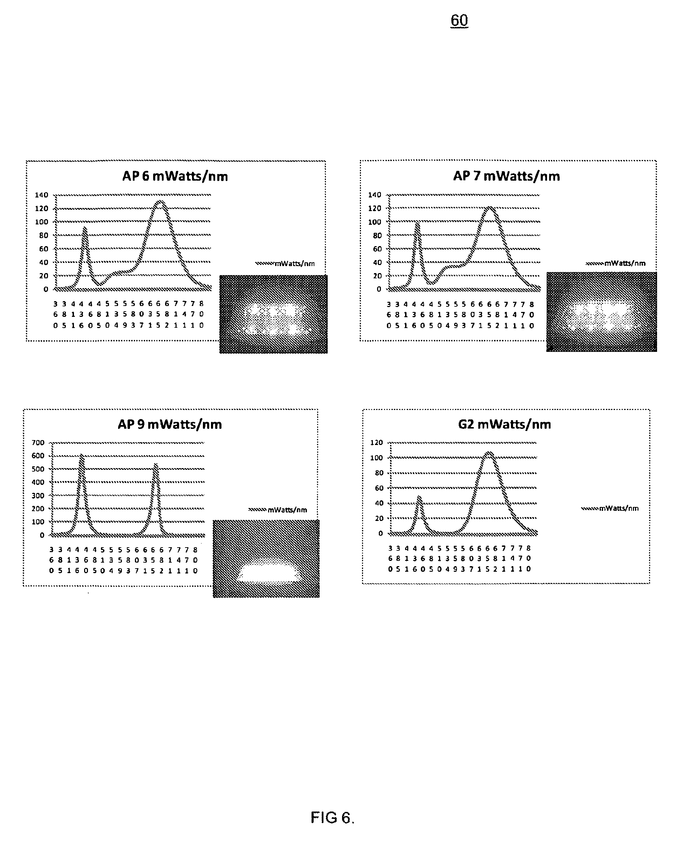

Figure 6 shows preferable LED spectra used in accordance with the invention that have been built and tested by the applicant. Wavelength is shown on the horizontal x-axis and relative intensity in the vertical y-axis. One preferable embodiment is known to be a UV LED + G2 LED of figure 6 or a UV LED and AP9 LED of the applicant from figure 6. Another preferred embodiment of the invention combines the spectra AP6 and AP7 of figure 6 with UV LEDs.

In one embodiment of the invention, the at least one UV LED or LEDs emit in the UV-A (315-400 nm) and UV-B (280-315 nm), but not in the UV C (100-280 nm) bands in accordance with the invention.

It should be noted that any advantageous dosage regime of artificial light to treat transplantation shock maybe applied in accordance with the invention. In one embodiment small exposures of artificial light are administered at known or random intervals. In other embodiments the light exposure to treat transplantation shock is administered at the end of the indoors growth period in accordance with the invention.

It should further be noted that it is in accordance with the invention to combine the artificial light treatment of the invention with other transplantation shock treatments, such as cooling of the seedlings. In some embodiments the artificial lights of the invention are housed in a refrigerator, so that seedlings are arranged to be acclimatised to outdoor winter conditions in the said refrigerator. Similarly, the artificial light treatment of transplantation shock, may be combined with artificial wind, or controlled diet of minerals for the seedlings in accordance with the invention.

It should further be noted that in any of the preceding embodiments of the invention the light device arranged to produce articial light for the treatment of transplantation shock may be equipped with any of the following phosphorescing materials:
- MAISiN₃X (where in M is Metal such as Ca, Sr, Ba and X is rare earth element such as Eu or Mn in any various ratios and combinations),
- MMgSiOX (where in M is Metal such as Ca, Sr, Ba and X is rare earth element such as Eu or Mn in any various ratios and combinations).

The invention has been explained above with reference to the aforementioned embodiments and several commercial and industrial advantages have been demonstrated. The methods and arrangements of the invention allow treating tree seedlings against transplantation shock, and thereby increase the likelihood of a successful planting of the tree seedling to the outdoors. The treatment of the invention reduces work phases for the grower, as the invention removes the need for sunshade curtains during nursing of the tree seedlings, and subsequently the need to move the seedlings to and from the shade area.

The invention has been explained above with reference to the aforementioned embodiments. However, it is clear that the invention is not only restricted to these embodiments, but comprises all possible embodiments within the scope of the inventive thought and the following patent claims.

### REFERENCES

"The physiological basis of containerised tree seedlings 'transplant shock': a review", Dugald C. Close, Christopher L Beadle and Philip H. Brown, Australian Forestry 2005, Vol. 68 No. 2 pp. 112-120.

EP 0364952 A2, Determining seed viability, Taylor et al., 1990.

US 2008/0120736, Process of photomorphogenically enhancing plants, William E. Hurst, 2008.

Photobiology of higher plants, Maurice S. McDonald, John Wiley & Sons, 2003.

"Effects of UV-B Radiation and Water Stress on Hardening Phase Growth of Container-Grown Betula platyphylla Seedlings", Jong Jin Kim and Sung Gak Hong Jour. Korean For. Soc. 87(4). 601 - 610. 1998.

## Claims

1. A method of treating plants, wherein
- at least one said plant is a tree seedling and is housed indoors (100),
- the said tree seedlings are exposed to artificial UV light indoors prior to outdoor life (110), **characterized in that**,
- incident UV light is produced by light emitting diodes (LEDs) (110) in both the UV-A (315-400 nm) and UV-B (280-315 nm) bands to treat transplantation shock in said plants.

2. A method as claimed in claim 1, **characterized in that**, artificial light is applied in small doses during the major part of nursing of at least one seedling (210, 310) and/or as a single period in the end of the in-doors nursing period.

3. A method as claimed in claim 1, **characterized in that**, artificial light (220, 320, 321) is applied in any of the following bands; violet and blue areas (400-500 nm), red and far red areas (600-800 nm) and/or green and yellow areas of the spectrum (500-600 nm).

4. A method as claimed in claim 1, **characterized in that**, artificial light spectrum (250) combined with the spectrum of Sun light transparent through a greenhouse wall and/or ceiling (240) amounts to a combined sum spectrum similar to the solar spectrum observed on the ground of the Earth with or without an intensity gap between 500-600 nm.

5. A method as claimed in claim 1, **characterized in that**, artificial light spectrum is similar to the solar spectrum observed on the ground of the Earth.

6. A method as claimed in claim 1, 4 and/or 5, **characterized in that**, green and yellow areas of the spectrum (500-600 nm) are omitted or have a smaller relative spectral intensity compared with blue or red bands than in sunlight.

7. A method as claimed in claim 4, **characterized in that**, the at least one tree seedling (210) is housed in an indoor greenhouse (200) with transparent walls.

8. A method as claimed in claim 5, **characterized in that**, the at least one tree seedling (310) is housed in a growth chamber (360).

9. A method as claimed in claim 1, **characterized in that**, the light device (220, 320, 321) is any of the following: a Light Emitting Diode (LED), AlInGaP red and AlInGaN green and/or blue HB-LEDs, a light wavelength up-conversion phosphorescing material which is deposited in direct proximity of the LED chip, europium-cerium co-doped BaₓSr_{y} ZnS₃, MAISiN₃X and/or MMgSiOX (where in M is Metal such as Ca, Sr, Ba and X is rare earth element such as Eu or Mn in any various ratios and combinations) -phosphors or -phosphorescing materials and/or cerium doped lanthanide oxide sulfides in direct proximity of the LED chip, and/or a wavelength up-conversion device by means of at least one semiconductor quantum dot, which is placed near the LED.

10. A method as claimed in any of the preceding claims, **characterized in that**, the at least one said tree seedling (210, 310) is exposed to the artificial light spectrum of claim 4 during the day.

11. A method as claimed in any of the preceding claims, **characterized in that**, the said at least one tree seedling (210, 310) is exposed to the artificial light spectrum of claim 5 during the night.

12. Use of artificial UV LED light in the UV-A and UV-B bands to treat transplantation shock in tree seedlings.

## Patentansprüche

1. Ein Verfahren zur Behandlung von Pflanzen, wobei
- wenigstens eine Pflanze ein Baumsämling ist und drinnen (100) untergebracht ist,
- die Baumsämlinge vor dem Leben draußen (110) drinnen künstlichem UV-Licht ausgesetzt werden, **dadurch gekennzeichnet, dass**
- einfallendes UV-Licht durch Leuchtdioden (LEDs) (110) in sowohl der UV-A- (315-400 nm) als auch UV-B- (280-315 nm) Bande produziert wird, um Verpflanzungsschock in den Pflanzen zu behandeln.

2. Ein Verfahren wie in Anspruch 1 beansprucht, **dadurch gekennzeichnet, dass** künstliches Licht in kleinen Dosen während des Großteils der Pflege des wenigstens einen Sämlings (210, 310) angewendet wird und/oder als eine einzige Periode am Ende der Pflegeperiode drinnen.

3. Ein Verfahren wie in Anspruch 1 beansprucht, **dadurch gekennzeichnet, dass** künstliches Licht (220, 320, 321) in irgendeiner der folgenden Banden angewendet wird: violette und blaue Bereiche (400-500 nm), rote und dunkelrote Bereiche (600-800 nm) und/oder grüne und gelbe Bereiche des Spektrums (500-600 nm).

4. Ein Verfahren wie in Anspruch 1 beansprucht, **dadurch gekennzeichnet, dass** künstliches Lichtspektrum (250) kombiniert mit dem Spektrum von Sonnenlicht, das durch die Wand und/oder Decke (240) eines Gewächshauses hindurch scheint, einem kombinierten Summenspektrum ähnlich dem am Erdboden beobachteten solaren Spektrum gleichkommt, mit oder ohne eine Intensitätslücke zwischen 500-600 nm.

5. Ein Verfahren wie in Anspruch 1 beansprucht, **dadurch gekennzeichnet, dass** künstliches Lichtspektrum dem am Erdboden beobachteten solaren Spektrum ähnlich ist.

6. Ein Verfahren wie in Anspruch 1, 4 und/oder 5 beansprucht, **dadurch gekennzeichnet, dass** grüne und gelbe Bereiche des Spektrums (500-600 nm) ausgelassen werden oder verglichen mit blauen oder roten Banden eine kleinere relative spektrale Intensität als in Sonnenlicht haben.

7. Ein Verfahren wie in Anspruch 4 beansprucht, **dadurch gekennzeichnet, dass** der wenigstens eine Baumsämling (210) in einem Innengewächshaus (200) mit transparenten Wänden untergebracht ist.

8. Ein Verfahren wie in Anspruch 5 beansprucht, **dadurch gekennzeichnet, dass** der wenigstens eine Baumsämling (310) in einer Wachstumskammer (360) untergebracht ist.

9. Ein Verfahren wie in Anspruch 1 beansprucht, **dadurch gekennzeichnet, dass** die Beleuchtungsvorrichtung (220, 320, 321) irgendeine der folgenden ist: eine Leuchtdiode (LED), AlInGaP rot und AlInGaN grün und/oder blaue HB-LEDs, ein Lichtwellenlängen-Hochwandlungs-phosphoreszierendes Material, das in direkter Nähe des LED-Chips abgelegt ist, Europium-Cerium ko-dotiertes BaₓSryZnS₃, MAlSiN₃X und/oder MMgSiOX (wobei M ein Metal ist, wie etwa Ca, Sr, Ba, und X ein seltene Erde-Element ist, wie etwa Eu oder Mn, in irgendwelchen verschiedenen Verhältnissen und Kombinationen) -Phosphore oder -phosphoreszierende Materialien und/oder Cerium-dotierte Lanthanoidoxidsulfide in direkter Nähe des LED-Chips und/oder eine Wellenlängen-Hochwandlungsvorrichtung durch wenigstens einen Halbleiter-Quantenpunkt, der nahe der LED platziert ist.

10. Ein Verfahren wie in irgendeinem der vorangehenden Ansprüche beansprucht, **dadurch gekennzeichnet, dass** der wenigstens eine Baumsämling (210, 310) während des Tages dem künstlichen Lichtspektrum des Anspruchs 4 ausgesetzt wird.

11. Ein Verfahren wie in irgendeinem der vorangehenden Ansprüche beansprucht, **dadurch gekennzeichnet, dass** der wenigstens eine Baumsämling (210, 310) während der Nacht dem künstlichen Lichtspektrum des Anspruchs 5 ausgesetzt wird.

12. Verwendung künstlichen UV-LED-Lichts in den UV-A- und UV-B-Banden zur Behandlung von Verpflanzungsschock in Baumsämlingen.

## Revendications

1. Procédé de traitement de plants, dans lequel
- au moins un desdits plants est un plant d'arbre et est logé dans un lieu fermé (100),
- lesdits plants d'arbre sont exposés à une lumière UV artificielle dans un lieu fermé avant leur vie à l'extérieur (110), **caractérisé en ce que**
- une lumière UV incidente est produite par des diodes électroluminescentes (DEL) (110) à la fois dans les bandes UV-A (315 nm à 400 nm) et UV-B (280 nm à 315 nm) pour traiter le choc de la transplantation chez lesdits plants.

2. Procédé selon la revendication 1, **caractérisé en ce qu'**une lumière artificielle est appliquée à petite dose pendant la majeure partie des soins d'au moins un plant (210, 310) et/ou pendant une seule période à la fin de la période de soins en intérieur.

3. Procédé selon la revendication 1, **caractérisé en ce qu'**une lumière artificielle (220, 320, 321) est appliquée dans l'une quelconque des bandes suivantes : les zones violettes et bleues (400 nm à 500 nm), les zones rouges et rouge lointain (600 nm à 800 nm) et/ou les zones vertes et jaunes du spectre (500 nm à 600 nm).

4. Procédé selon la revendication 1, **caractérisé en ce qu'**un spectre de lumière artificielle (250) combiné avec le spectre de la lumière solaire transparent à travers une paroi et/ou un plafond (240) d'une serre équivaut à un spectre total combiné similaire au spectre solaire observé sur le sol de la Terre, avec ou sans écart d'intensité entre 500 nm et 600 nm.

5. Procédé selon la revendication 1, **caractérisé en ce que** le spectre de lumière artificielle est similaire au spectre solaire observé sur le sol de la Terre.

6. Procédé selon la revendication 1, 4 et/ou 5, **caractérisé en ce que** les zones vertes et jaunes du spectre (500 nm à 600 nm) sont omises ou présentent une intensité spectrale relative plus petite, en comparaison avec les bandes bleues ou rouges, que dans la lumière solaire.

7. Procédé selon la revendication 4, **caractérisé en ce que** l'au moins un plant d'arbre (210) est logé dans une serre d'intérieur (200) ayant des parois transparentes.

8. Procédé selon la revendication 5, **caractérisé en ce que** l'au moins un plant d'arbre (310) est logé dans une chambre de croissance (360).

9. Procédé selon la revendication 1, **caractérisé en ce que** le dispositif lumineux (220, 320, 321) est l'un quelconque des suivants : une diode électroluminescente (DEL), des DEL haute brillance rouges à AlInGaP et bleues et/ou vertes à AlInGaN, un matériau phosphorescent à conversion par élévation de la longueur d'onde de la lumière qui est déposé à proximité immédiate de la puce à DEL, des substances luminescentes ou phosphorescentes à base de BaₓSr_{y}ZnS₃, MalSiN₃X et/ou MMgSiOX (où M est un métal, tel que Ca, Sr, Ba, et X est un lanthanide, tel que Eu ou Mn, dans divers rapports et diverses combinaisons) co-dopé avec de l'europium-cérium et/ou des sulfures d'oxyde de lanthanide dopés au cérium à proximité immédiate de la puce à DEL, et/ou un dispositif de conversion par élévation de la longueur d'onde au moyen d'au moins un point quantique semiconducteur, qui est placé à proximité de la DEL.

10. Procédé selon l'une quelconque des revendications précédentes, **caractérisé en ce que** ledit au moins un plant d'arbre (210, 310) est exposé au spectre de lumière artificielle de la revendication 4 pendant le jour.

11. Procédé selon l'une quelconque des revendications précédentes, **caractérisé en ce que** ledit au moins un plant d'arbre (210, 310) est exposé au spectre de lumière artificielle de la revendication 5 pendant la nuit.

12. Utilisation d'une lumière UV artificielle de DEL dans les bandes UV-A et UV-B pour traiter le choc de la transplantation chez des plants d'arbre.
